# FASCICULE DE BREVET EUROPEEN

(11) **EP 0 721 500 B1**
(45) Date de publication et mention de la délivrance du brevet: **20.07.2005**
(21) Numéro de dépôt: 94928920.1
(22) Date de dépôt: 27.09.1994
(51) Int. Cl.: C12N 5/10, C12N 5/00, A61F 2/02

(54) **LIGNEES DE CELLULES SECRETRICES D'INSULINE, PROCEDES D'OBTENTION ET UTILISATION**
INSULIN SEKRETIERENDE ZELLINIE, VERFAHREN ZUR HERSTELLUNG, UND DEREN VERWENDUNG
INSULIN-SECRETING CELL LINES, METHODS OF PRODUCTION AND USE

(30) Priorité: 30.09.1993 FR 9311687
(43) Date de publication de la demande: 17.07.1996
(73) Titulaire: MERCK PATENT GmbH, 64293 Darmstadt (DE)
(72) Inventeur: ASFARI, Maryam, F-75015 Paris (FR); CZERNICHOW, Paul, F-75020 Paris (FR)
(86) Numéro de dépôt international: PCT/FR1994/001129
(87) Numéro de publication internationale: WO 1995/009231

(56) Documents cités:
- DE-A- 4 229 526
- METHODS IN ENZYMOLOGY, vol.101, 1983 pages 202 - 211 RODNEY J. ROTHSTEIN 'ONE-STEP GENE DISRUPTION IN YEAST'
- BIOLOGICAL ABSTRACTS, vol. 93 1992, Philadelphia, PA, US; abstract no. 132998, ASFARI,MARYAM ET AL. 'ESTABLISHMENT OF 2-MERCAPTOETHANOL-DEPENDENT DIFFERENTIATED INSULIN-SECRETING CELL LINES' & ENDOCRINOLOGY, vol.130, no.1, 1992 pages 167 - 178

## Description

La présente invention se rapporte au domaine de la biologie et plus particulièrement au domaine de la biologie cellulaire.

Elle a plus particulièrement pour objet, une lignée cellulaire nouvelle, susceptible d'être implantée dans un organe humain pour lui faire exprimer le produit biologique que la lignée cellulaire nouvelle exprime normalement dans les cultures.

L'invention a spécifiquement pour objet, une nouvelle lignée cellulaire dite lignée cellulaire β répondant au glucose (INS-1) dont la principale propriété est la sensibilité au glucose. Ces cellules sont donc caractérisées par un contenu élevé en insuline, la possibilité d'exprimer de la glucokinase et du transporteur de glucose Glut 2 à des niveaux comparables à ceux des cellules β-normales et, en outre, ces cellules sont rendues non proliférantes par manipulation génétiques.

Cette lignée cellulaire est donc apte à être greffée dans les organes des sujets insulino-dépendants et d'assurer un contrôle "physiologique" de la glycémie dans le cas de diabète insulino-dépendant.

La greffe d'organe ou la greffe de tissu fait partie des outils thérapeutiques utilisés dans un certain nombre de maladies. Plus récemment, les techniques de manipulation génétique, ont fait entrevoir d'autres possibilités de traitement des maladies jusque-là incurables.

On propose donc un projet de recherche qui combine la greffe et la thérapie génique pour le traitement du diabète sucré. En effet, les Demandeurs ont établi une lignée cellulaire β, la lignée INS-1. Cette lignée sécrète l'insuline en réponse à des concentrations physiologiques du glucose et pourrait, après manipulation génétique, être utilisée pour la greffe et le contrôle "physiologique" de la glycémie dans le diabète insulino-dépendant.

Malgré les efforts thérapeutiques réalisés dans la dernière décennie, le traitement du diabète reste très imparfait. C'est la raison pour laquelle des thérapeutiques nouvelles sont explorées; les greffes du pancréas ou d'îlots en font partie (Hellerstrom C, Andersson A, Groth C-G, Sandler S, Jansson L, Korsgren O, Svenne I, Petersson B, Tollemar J, Tydén G - Diabetes Care 11 (Suppl.1) 45-53 1988) (Pipelleers DG, Pipelleers-Marichal M, Bannaert J-C, Berghmans M, In't Veld PA, Rozin J, Van de Winkel M, Gepts W - Diabetes 40:908-919 1991). Plus d'un millier de greffe de pancréas entier et quelques dizaines de greffe d'îlots ont été réalisées de par le monde. Bien que porteuses d'espérance, ces techniques lourdes soulèvent schématiquement deux types de problèmes : les uns immunologiques, les autres logistiques.

En plus des problèmes de tolérance inhérents à chaque type de greffe, il faut rappeler que le diabète est une maladie auto-immune et que le receveur, diabétique, garde une potentialité à détruire les cellules β greffées. Il y a donc une nécessité absolue d'associer une thérapeutique immuno-suppressive à la greffe. Afin d'éviter cette immuno-suppression, certains auteurs ont proposé la transplantation d'îlots encapsulés (Lacy P, Hegre OD, Gerasimidi-Vazeo A, Gentile FT, Dionne KE - Science 254:1782-1784 1991) (Chicheportich D, Reach G - Diabetologia 31:54-57 1988). L'avantage essentiel de la protection et notamment d'encapsulation est de soustraire le tissu greffé à l'attaque par le système immunitaire. Toutefois, la faible disponibilité des îlots rend difficile la préparation de quantités suffisantes du matériel dans des buts thérapeutiques. C'est pourquoi des efforts considérables ont été consacrés ces dix dernières années pour établir des lignées cellulaires β comme modèles d'étude de l'insulinosecrétion et du diabète. La plupart de ces lignées ont perdu leur fonction cellulaire β essentielle, à savoir la réponse au glucose par une secrétion d'insuline, cette perte survenant au cours des repiquages et des cycles cellulaires successifs. Les Demandeurs ont donc pu établir une lignée cellulaire β très différenciée (INS-1) dont les caractéristiques sont très proches de celles des cellules β normales (Asfari M, Janjic D, Meda P, Li G, Halban PA, Wollheim CB - Endocrinology 130:167-178 1992) et ceci constitue l'essence de l'invention.

Parmi les propriétés remarquables de ces cellules, on citera en particulier, le contenu en insuline élevé, l'expression de la glucokinase, du transporteur de glucose Glut 2, à des niveaux comparables à ceux de la cellule β normale. Enfin, la réponse à des facteurs de croissance ou de différenciation comme l'hormone de croissance, la prolactine IGF-1 et IGF-II à des concentrations physiologiques. L'intérêt essentiel de ces cellules est avant tout leur sensibilité au glucose. Les Demandeurs ont pu montrer précédemment qu'une augmentation des concentrations de glucose de 3 à 20 mM augmente de 4 fois la secrétion d'insuline des cellules INS-I en présence de substances qui élèvent le taux intracellulaire de AMP_{c}.

Récemment, il a été de plus montré que l'incubation de ces cellules avec des concentrations physiologiques de glucose (48h à 5 mM) les rend encore plus sensibles à des variations de concentration de glucose. Ces caractéristiques de différenciation font de ces cellules, les candidates les plus appropriées pour des xénotransplantations dans des capsules biocompatibles chez des sujets diabétiques.

Cependant, avant la greffe, il est nécessaire de rendre ces cellules non proliférantes. En effet, l'inhibition de la prolifération est indispensable si l'on veut éviter une croissance anarchique et une éventuelle destruction de la capsule protectrice. Ceci est obtenu par destruction des gènes impliqués (directement ou indirectement) dans la prolifération cellulaire. On a pu montrer récemment que l'IGF-II est abondamment exprimé dans ces cellules. L'IGF-II est un facteur de croissance qui module la croissance cellulaire essentiellement de manière autocrine. On peut donc penser, sur la base des résultats récents qui sont décrits plus loin, que l'IGF-II est impliqué dans la croissance incontrôlée de ces cellules et que l'inhibition de l'expression du gène de l'IGF-II (par destruction du gène) est susceptible d'inhiber ou de diminuer considerablement la prolifération cellulaire. Par cette manipulation génétique, les cellules INS-I seraient en mesure d'être adaptées à l'encapsulation et à la transplantation en permettant d'éviter toutes les complications et les difficultés liées à la préparation et la transplantation d'îlots pancréatiques.

Finalement, la prolifération des cellules β, modifiées génétiquement, cultivées en l'absence de sérum, est réduite de manière considérable lorsque les cellules sont cultivées en présence de protéine de liaison IGFBP-3 (Gopinath R, Walton PE, Etherton ED - Endocrinol 120:231-236 1989). Dans le modèle IGFBP-3, mis au point par les Demandeurs, sequestrant IGF-II, on diminue son activité biologique. L'addition d'IGFBF inhibe la prolifération des cellules INS-I. Cette observaton suggère que l'IGF-II joue un rôle majeur dans la prolifération des cellules INS-I car la seule fonction connue des protéines de liaison (BP) et en particulier BP3, est de se lier aux IGF.

Un certain nombre de preuves indirectes sont en faveur des résultats obtenus : IGF-II stimule la prolifération cellulaire dans les lignées tumorales de manière autocrine-paracrine (El-Bardy OM, Romanus JAI, Helman LH, Cooper MH, Rrchler MM, Israel MA - J. Clin. Invest. 84:829 1989). Ceci aussi a été démontré sur des cellules transformées (Park JHY, McCusker RH, Vanderhoof JA, Mohammadpour H, Harty RF, MacDonald RG - Endocrinology 131:1359-1368 1992) et sur des cultures d'îlots β primaires (Rabinovith A, Quigley C, Russel T, Patel Y, Mintz DH - Diabetes 31:160-164 1982) (Hill DJ, Hogg J - E.M Spencer (ed), Elsvier Science publishing CO, INC 1991 235-240).

L'invention a également pour objet, l'établissement de lignées cellulaires INS-I, modifiées génétiquement, encapsulées, en vue de leur implantation.

### RESULTATS PRELIMINAIRES

Ces résultats concernent essentiellement les relations entre l'IGF-II, la concentration de glucose dans le milieu de culture et la prolifération cellulaire.

A 5 mM de glucose, la prolifération cellulaire ne semble pas modifiée. A partir de 5 mM jusqu'à 20 mM, on observe une augmentation de la prolifération pendant des périodes allant jusqu'à 72 h de culture. En parallèle, on observe une augmentation des ARN messagers d'IGF-II pour des concentrations de glucose allant de 10 à 20 mM.

On n'a pas pu détecter ni la protéine IGF-I (radioimmunoessai)(Binoux M, Seurin D, Lassarre C, Gourmelen M - J. Clin. Endocrinol. Metab. 59:453-462 1984), ni son ARN messager dans ces cellules, même après de longues expositions à l'hormone de croissance.

### PRINCIPE DE LA METHODE

Le travail devra se dérouler en deux étapes :
- établir une lignée de cellules INS-I porteuse d'un gène d'IGF-II inactif, rendant les cellules non prolifératives tout en maintenant leur sensibilité au glucose
- encapsulation de ces cellules et transplantation chez les animaux diabétiques dans la perspective de réaliser, ultérieurement, des greffes chez l'homme selon le même mode opératoire.

### PROTOCOLE EXPERIMENTAL

### Partie I :

L'inactivation permanente du gène d'IGF-II a été réalisée par la technique de recombinaison homologue (Riete H, Maandag ER, Clarke A, Hooper M, Berns A - Nature 384:649-651 1990) (Pennington S, Wilson JH - Proc. Natl. Acad.Sci. 88:9498-9502 1991). Les Demandeurs ont à leur disposition le gène cloné d'IGF-II dans le plasmide pUC 119 (Dr HOLTHUIZEN)(Holthuizen P, Van der Lee FM, Ikejiri K, Yamamoto M, Sussenbach JS - Biochem Biophys. Acta 1087:341-343 1990). Parmi les quatre différents types d'ARN messager codant pour l'IGF-II, principalement l'ARN messager de 3,6kb est exprimé dans les cellules INS-I. Ce messager est transcrit à partir du promoteur P3 (Matsuguchi T, Takahashi K, Ikejiri K, Ueno T, Endo H, Yamamoto M - Biochem Biophys Acta 1048:165-170 1990).

On se propose d'insérer une cassette codant pour le gène de résistance à la néomycine (néo) sous contrôle du promoteur des gènes précoces du CMV, placé en phase dans l'exon 4 du gène de l'IGF-II. En aval de cette construction, une cassette codant pour la thymidine-kinase du virus de l'herpès simplex sera insérée. Cette construction permet de sélectionner les rares clônes ayant la construction IGF-II-néo par recombinaison homologue et non par insertion au hasard. En effet, les clônes dûs à une recombinaison homologue seront résistants à la néomycine et au gancyclovir alors que les clônes dûs à une recombinaison au hasard seront néomycino-résistants mais gancyclovir sensibles.

Les cellules, après transfection, sont cultivées en présence de néomycine, de gancyclovir et d'IGF-II. Les clônes résistants sont testés pour leur capacité à proliférer en l'absence d'IGF-II endogène mais en présence d'IGF-II exogène et seront étudiés par la technique du Southern Blot et analysés par PCR dans le but de contrôler si l'intégration a été correcte. L'expression d'IGF-II sera étudiée au niveau des ARN messagers et des protéines par les techniques de Northern et Western-Blot respectivement. Puisqu'il est établi que, seul l'allèle paternel du gène d'IGF-II est actif (De Chaiara TM, Robertson EJ, Efstratiadis A - Cell 64:849-859 1991), et puisque la recombinaison homologue est plus efficace au niveau des segments d'ADN, transcriptionnellement actifs (Nickoloff JC - MEB 12:5311-5318 1992), on peut espérer obtenir des cellules INS-I n'exprimant plus IGF-II par une seule étape de transfection/sélection. Toutefois, si ça n'était pas le cas, la destruction de la deuxième copie du gène IGF-II peut être entreprise (Riete H, Maandag ER, Clarke A, Hooper M, Berns A - Nature 384:649-651 1990). A chaque étape de ce travail, le pouvoir du glucose à induire la sécrétion d'insuline doit être vérifié.

### Partie II :

Si la destruction ("knock-out") du gène d'IGF-II est réussie, on doit finalement obtenir une lignée cellulaire INS-I, qui sera entièrement ou en grande partie dépendante de l'administration exogène d'IGF-II pour sa croissance. Théoriquement, cette nouvelle lignée cellulaire sera sensible au glucose de la même manière que la lignée parentale. Dans ce cas, ces cellules pourront donc être utilisées pour l'encapsulation et la transplantation.

### 1. Encapsulation de la lignée cellulaire INS-I

a) pour une encapsulation efficace, les cellules doivent être aggregées dans une forme de "pseudo-îlots". Ceci peut être effectué par culture des cellules dans des boites de culture non-adhésives. La taille des agregats (="pseudo-îlots") peut être déterminée par la densité des cellules au moment de la mise en culture. D'autre part, le nombre de cellules dans les aggregats peut être vérifié après trypsination des "pseudo-îlots" (PI) récoltés.
b) un nombre connu de PI contenant une quantité d'insuline équivalente à celle trouvée dans 500-1000 îlots de rats adultes (2-4 x 10⁸ cellules) est mis en suspension dans une solution d'alginate de sodium (Lacy P, Hegre OD, Gerasimidi-Vazeo A, Gentile FT, Dionne KE - Science 254:1782-1784 1991) (Lanza RP, Butler DH, Borland KM, Staruk JE, Faustman DL, Solomon BA, Muller TE, Rupp RG, Maki T, Monaco AP, Chick WL - Proc. Natl. Acad. Sci 88:11100-11104 1991) et immobilisé par encapsulation dans cet hydrogel biocompatible par une solution aqueuse d'un sel de Calcium. Les PI encapsulés seront ensuite placés dans des membranes tubulaires acryliques perméables à des molécules de poids moléculaire inférieur à 50 000-80 000 (Lacy P, Hegre 0D, Gerasimidi-Vazeo A, Gentile FT, Dionne KE - Science 254:1782-1784 1991) (Lanza RP, Butler DH, Borland KM, Staruk JE, Faustman DL, Solomon BA, Muller TE, Rupp RG, Maki T, Monaco AP, Chick WL - Proc. Natl. Acad. Sci 88:11100-11104 1991).
c) les PI encapsulés sont étudiés "in vitro" par perifusion pour déterminer avec quelle intensité et à quelle vitesse ils provoquent un changement de sécrétion d'insuline en réponse à des concentrations de glucose variées. Lorsque cette partie de l'expérience se montre satisfaisante, les PI contenus dans des fibres seront utilisés pour la transplantation.

### 2. Transplantation

Les PI encapsulés sont transplantés par voie sous-cutanée ou intra-péritoneale. Les deux méthodes sont efficaces dans le maintien de la normoglycémie chez les modèles animaux du diabète insulino-dépendant humain. Les rats Bio-breeding (BB) et les souris non-obèses diabétiques (NOD) qui développent spontanément un diabète sont associés à un génotype particulier d'histocompatibilité et présentent les mêmes défauts sur le plan immunitaire que les sujets diabétiques (présence des lymphocytes T cytotoxiques) des animaux diabétiques transplantés (Lacy P, Hegre OD, Gerasimidi-Vazeo A, Gentile FT, Dionne KE - Science 254:1782-1784 1991) (Lanza RP, Butler DH, Borland KM, Staruk JE, Faustman DL, Solomon BA, Muller TE, Rupp RG, Maki T, Monaco AP, Chick VL - Proc. Natl. Acad. Sci 88:11100-11104 1991). La transplantation par voie intra-péritonéale parait la plus favorable pour assurer un bon environnement biologique aux cellules greffées.

Cette technique est applicable de la même façon aux sujets insulino-dépendants. Il faut implanter entre 50 000 et 200 000 PI pour atteindre une concentration efficace d'insuline.

## Revendications

1. Lignée cellulaire β insulinosécrétrice hautement différentiée comprenant des cellules β transformées, dans laquelle lesdites cellules β expriment de l'insuline en réponse au glucose, et dans laquelle l'expression du gène du facteur de croissance insulinoïde de type II (IGF-II) dans lesdites cellules β est inhibé de façon permanente; ladite lignée cellulaire β est obtenable par une méthode comprenant les démarches suivants:
(i) la transfection de cellules β d'une lignée cellulaire β insulinosécrétrice hautement différentiée par un plasmide portant un gène IGF-II modifié, dans lequel on a inséré dans ledit gène IGF-II modifié, en phase dans l'exon 4, une cassette codant pour le gène de résistance à la néomycine, et, en aval dudit gène de néomycine, une cassette codant pour la thymidine-kinase virale,
(ii) la mise en culture desdites cellules β transfectées en présence de néomycine, de gancyclovir et d'IGF-II, et
(iii) l'isolement des cellules β transformées qui sont résistantes à la néomycine et au gancyclovir, ce par quoi on obtient des cellules β transformées,
dans laquelle ledit gène IGF-II modifié a été incorporé par une recombinaison homologue, ce par quoi lesdites cellules β sont dépendantes de l'administration exogène d'IGF-II pour leur prolifération

2. Lignée cellulaire selon la revendication 1, dans laquelle l'expression du gène du facteur de croissance insulinoïde de type II (IGF-II) dans ladite lignée cellulaire β est totalement réprimé.

3. Lignée cellulaire β selon la revendication 1, dans laquelle la lignée cellulaire β insulinosécrétrice hautement différentiée est une lignée cellulaire INS-I.

4. Lignée cellulaire β selon la revendication 1, dans laquelle ladite lignée cellulaire β est sous la forme d'agrégats de pseudo-îlots.

5. Méthode de production d'une lignée cellulaire β transformée selon une des revendications 1 - 4, comprenant
(i) la transfection de cellules β d'une lignée cellulaire β insulinosécrétrice hautement différentiée par un plasmide portant un gène IGF-II modifié, dans lequel on a inséré dans ledit gène IGF-II modifié, en phase dans l'exon 4, une cassette codant pour le gène de résistance à la néomycine, et, en aval dudit gène de néomycine, une cassette codant pour la thymidine-kinase virale,
(ii) la mise en culture desdites cellules β transfectées en présence de néomycine, de gancyclovir et d'IGF-II, et
(iii) l'isolement des cellules β transformées qui sont résistantes à la néomycine et au gancyclovir, ce par quoi on obtient des cellules β transformées,
dans laquelle ledit gène IGF-II modifié a été incorporé par une recombinaison homologue, ce par quoi lesdites cellules β sont dépendantes de l'administration exogène d'IGF-II pour leur prolifération.

6. Méthode selon la revendication 5, dans laquelle la lignée cellulaire β insulinosécrétrice hautement différentiée est une lignée cellulaire INS-I.

7. Méthode selon la revendication 5 ou 6, dans laquelle le plasmide est dérivé de pUC 119.

8. Méthode selon une des revendications 5 - 7, dans laquelle la lignée cellulaire β ainsi produite est transformée davantage en agrégats sous la forme de pseudo-îlots par la mise en culture des cellules dans des boîtes de culture non adhérentes.

9. Méthode selon la revendication 8, dans laquelle les pseudo-îlots sont agglomérés en suspension dans un agent gélifiant et immobilisés dans un hydrogel biocompatible par l'addition d'un agent d'immobilisation.

10. Méthode selon la revendication 9, dans laquelle l'agent gélifiant est un alginate de sodium.

11. Méthode selon la revendication 9 ou 10, dans laquelle l'agent d'immobilisation est une solution aqueuse d'un sel de calcium.

12. Méthode selon une des revendications 9 - 11, dans laquelle les pseudo-îlots agglomérés et immobilisés contiennent une quantité d'insuline équivalente à celle trouvée dans de 500 à 1000 îlots chez le rat adulte.

13. Méthode selon une des revendications 9 - 12, dans laquelle les pseudo-îlots immobilisés par encapsulation dans un hydrogel biocompatible qui a été durci par l'addition d'une solution aqueuse d'un sel de calcium, sont ensuite placés dans des membranes tubulaires acryliques, qui sont perméables à des molécules ayant un poids moléculaire inférieur à 50.000-80.000.

14. Méthode selon la revendication 13, dans laquelle les pseudo-îlots immobilisés et durcis sont incorporés dans des fibres transplantables.

15. Dispositif transplantable biocompatible, comprenant des pseudo-îlots d'une lignée cellulaire β insulinosécrétrice transformée selon une des revendications 1 - 4, agglomérés et incorporés dans des membranes tubulaires acryliques qui sont perméables à des molécules ayant un poids moléculaire inférieur à 50.000-80.000, et distribués sous forme de fibres.

16. Dispositif transplantable selon la revendication 15, comprenant de 50.000 à 200.000 pseudo-îlots, ce par quoi une quantité efficace d'insuline pour le traitement d'un patient insulino-dépendant est produite.

## Patentansprüche

1. Hochdifferenzierte, insulinsezernierende β-Zelllinie, die transformierte β-Zellen umfasst, wobei die β-Zellen Insulin in Antwort auf Glucose exprimieren und wobei die Expression des Gens des insulinähnlichen Wachstumsfaktors Typ II (IGF-II) in den β-Zellen permanent inhibiert ist; wobei die β-Zelllinie durch ein Verfahren erhalten werden kann, das die folgenden Stufen umfasst:
(i) die Transfektion von β-Zellen einer hochdifferenzierten insulinsezernierenden β-Zelllinie mit einem Plasmid, das ein modifiziertes IGF-II-Gen trägt, wobei in das modifizierte IGF-II-Gen im Leserahmen in das Exon 4 eine Kassette, die das Gen für Neomycinresistenz codiert, und stromabwärts des Neomycingens eine Kassette, die für virale Thymidinkinase codiert, inseriert worden ist,
(ii) das Kultivieren der transfizierten β-Zellen in Gegenwart von Neomycin, Ganciclovir und IGF-II und
(iii) die Isolation der transfizierten β-Zellen, die gegen Neomycin und Ganciclovir resistent sind, wodurch transformierte β-Zellen erhalten werden,
wobei das modifizierte IGF-II-Gen durch homologe Rekombination eingebracht wurde, wodurch die β-Zellen von der exogenen Verabreichung von IGF-II abhängig werden, damit sie proliferieren.

2. Zelllinie nach Anspruch 1, wobei die Expression des Gens des insulinahnlichen Wachstumsfaktors Typ II (IGF-II) in der β-Zelllinie vollständig reprimiert ist.

3. β-Zelllinie nach Anspruch 1, wobei die hochdifferenzierte insulinsezernierende β-Zelllinie eine INS-I-Zelllinie ist.

4. β-Zelllinie nach Anspruch 1, wobei die β-Zelllinie in Form von Aggregaten von Pseudo-Inseln vorliegt.

5. Verfahren zur Herstellung einer transformierten β-Zelllinie nach einem der Ansprüche 1-4, umfassend
(i) die Transfektion von β-Zellen einer hochdifferenzierten insulinsezernierenden β-Zelllinie mit einem Plasmid, das ein modifiziertes IGF-II-Gen trägt, wobei in das modifizierte IGF-II-Gen im Leserahmen in das Exon 4 eine Kassette, die das Gen für Neomycinresistenz codiert, und stromabwärts des Neomycingens eine Kassette, die für virale Thymidinkinase codiert, inseriert worden ist,
(ii) das Kultivieren der transfizierten β-Zellen in Gegenwart von Neomycm, Ganciclovir und IGF-II und
(iii) die Isolation der transformierten β-Zellen, die gegen Neomycin und Ganciclovir resistent sind, wodurch transformierte β-Zellen erhalten werden,
wobei das modifizierte IGF-II-Gen durch homologe Rekombination eingebracht wurde, wodurch die β-Zellen von der exogenen Verabreichung von IGF-II abhängig werden, damit sie proliferieren.

6. Verfahren nach Anspruch 5, wobei die hochdifferenzierte insulinsezernierende β-Zelllinie eine INS-I-Zelllinie ist.

7. Verfahren nach Anspruch 5 oder 6, wobei das Plasmid ein Derivat von pUC 119 ist.

8. Verfahren nach einem der Ansprüche 5 - 7, wobei die so hergestellte β-Zelllinie ferner in Aggregaten in Form von Pseudo-Inseln transformiert wird, indem nicht angeheftete Zellen in Kulturflaschen kultiviert werden.

9. Verfahren nach Anspruch 8, wobei die Pseudo-Inseln in Suspension in einem Geliermittel agglomeriert und in einem biologisch verträglichen Hydrogel durch Zugabe eines Immobilisierungsmittels immobilisiert werden.

10. Verfahren nach Anspruch 9, wobei das Geliermittel ein Natriumalginat ist.

11. Verfahren nach Anspruch 9 oder 10, wobei das Immobilisierungsmittel eine wässrige Lösung eines Calciumsalzes ist.

12. Verfahren nach einem der Ansprüche 9 - 11, wobei die agglomerierten und immobilisierten Pseudo-Inseln eine Menge an Insulin enthalten, die äquivalent zu derjenigen ist, die man in 500 bis 1000 Inseln bei einer adulten Ratte findet.

13. Verfahren nach einem der Ansprüche 9 - 12, wobei die durch Einkapseln in ein biologisch verträgliches Hydrogel, das durch Zugabe einer wässrigen Lösung eines Calciumsalz gehärtet wurde, immobilisierten Pseudo-Inseln anschließend in röhrenförmige Acrylmembranen eingebracht werden, die für Moleküle mit einem Molekulargewicht von weniger als 50000 bis 80000 permeabel sind.

14. Verfahren nach Anspruch 13, wobei die immobilisierten und gehärteten Pseudo-Inseln in transplantierbare Fasern eingebracht werden.

15. Transplantierbare biologisch-verträgliche-Vorrichtung, die Pseudo-Inseln einer transformierten insulinsezernierenden β-Zelllinie nach einem der Ansprüche 1 - 4 umfasst, die agglomenert und in röhrenförmige Acrylmembranen eingebracht werden, die für Moleküle mit einem Molekulargewicht von weniger als 50000 bis 80000 permeabel und in Form von Fasern verteilt sind.

16. Transplantierbare Vorrichtung nach Anspruch 15, umfassend 50000 bis 200000 Pseudo-Inseln, wodurch eine wirksame Menge an Insulin zur Behandlung eines insulinabhängigen Patienten produziert wird.

## Claims

1. Highly differentiated, insulin-secreting β cell line comprising transformed β cells, in which said β cells express insulin in response to glucose, and in which the expression of the gene of type II insulinoid growth factor (IGF-II) in said β cells is permanently inhibited, said β cell line being obtainable by a process comprising the following steps:
(i) the transfection of β cells of a highly differentiated, insulin-secreting β cell line by a plasmid carrying a modified IGF-II gene, in which a cassette encoding for the neomycin resistance gene has been inserted into said modified IGF-II gene in phase in exon 4, and a cassette encoding for viral thymidine kinase has been inserted downstream of said neomycin gene,
(ii) the culture of said transfected β cells in the presence of neomycin, ganciclovir and IGF-II, and
(iii) the isolation of the transformed β cells that are resistant to neomycin and ganciclovir, whereby transformed β cells are obtained,
in which process said modified IGF-II gene has been incorporated by a homologous recombination, whereby said β cells are dependent on the exogenous administration of IGF-II for their proliferation.

2. Cell line according to Claim 1 in which the expression of the gene of the type II insulinoid growth factor (IGF-II) in said β cell line is totally supressed.

3. β cell line according to Claim 1 in which the highly differentiated, insulin-secreting β cell line is an INS-I cell line.

4. β cell line according to Claim 1 in which said β cell line is in the form of aggregates of pseudo-islets.

5. Process for the production of a transformed β cell line according to one of Claims 1 - 4, comprising
(i) the transfection of β cells of a highly differentiated, insulin-secreting β cell line by a plasmid carrying a modified IGF-II gene, in which a cassette encoding for the neomycin resistance gene has been inserted into said modified IGF-II gene in phase in exon 4, and a cassette encoding for viral thymidine kinase has been inserted downstream of said neomycin gene,
(ii) the culture of said transfected β cells in the presence of neomycin, ganciclovir and IGF-II, and
(iii) the isolation of the transformed β cells that are resistant to neomycin and ganciclovir, whereby transformed β cells are obtained,
in which process said modified IGF-II gene has been incorporated by a homologous recombination, whereby said β cells are dependent on the exogenous administration of IGF-II for their proliferation.

6. Process according to Claim 5 in which the highly differentiated, insulin-secreting β cell line is an INS-I cell line.

7. Process according to Claim 5 or 6 in which the plasmid is derived from pUC 119.

8. Process according to one of Claims 5 - 7 in which the β cell line produced in this way is transformed more into aggregates in the form of pseudo-islets by cultunng the cells in non-adhering culture dishes.

9. Process according to Claim 8 in which the pseudo-islets are agglomerated in suspension in a gelling agent and immobilised in a biocompatible hydrogel by adding an immobilising agent.

10. Process according to Claim 9 in which the gelling agent is a sodium alginate.

11. Process according to Claim 9 or 10 in which the immobilising agent is an aqueous solution of a calcium salt.

12. Process according to one of Claims 9 - 11 in which the agglomerated and immobilised pseudo-islets contain an amount of insulin equivalent to that found in 500 to 1000 islets in the adult rat.

13. Process according to one of Claims 9 - 12 in which the pseudo-islets immobilised by encapsulation in a biocompatible hydrogel that has been hardened by adding an aqueous solution of a calcium salt are then placed in acrylic tubular membranes permeable to molecules having a molecular weight below 50,000-80,000.

14. Process according to Claim 13 in which the immobilised and hardened pseudo-islets are incorporated into transplantable fibres.

15. Biocompatible transplantable device comprising pseudo-islets of a transformed insulin-secreting β cell line according to one of Claims 1 - 4, which are agglomerated and incorporated into acrylic tubular membranes permeable to molecules having a molecular weight below 50,000-80,000, and are distributed in the form of fibres.

16. Transplantable device according to Claim 15 comprising from 50,000 to 200,000 pseudo-islets, whereby an effective amount of insulin for treating an insulin-dependent patient is produced.
